# EUROPEAN PATENT SPECIFICATION

(11) **EP 0 934 929 B1**
(45) Date of publication and mention of the grant of the patent: **17.09.2003**
(21) Application number: 99300815.0
(22) Date of filing: 03.02.1999
(51) Int. Cl.: C07D 207/34, C07C 251/24

(54) **Process for the preparation of 2-aryl-5-(perfluoroalkyl)pyrrole compounds from N-(arylmethylene)-1-chloro-1-(perfluoroalkyl) methylamine compounds**
Verfahren zur Herstellung von 2-Aryl-5-(perflouralkyl)pyrrolderivaten aus N-(Arylmethylen)-1-chlor-1-(perfluoroalkyl)methylaminderivaten
Procédé pour la préparation de dérivés de 2-aryl-5-(perfluoroalkyl)pyrrole a partir de dérivés de n-(arylmethylene)-1-chloro-1-(perfluoroalkyl) methylamine

(30) Priority: 09.02.1998 US 20611
(43) Date of publication of application: 11.08.1999
(73) Proprietor: BASF AKTIENGESELLSCHAFT, 67056 Ludwigshafen (DE)
(72) Inventor: Kameswaran, Venkataraman, Trenton, New Jersey 08638 (US)
(74) Representative: Pohl, Michael, Dr.

(56) References cited:
- US-A- 5 145 986
- TANAKA K ET AL: "Isomerization of trifluoroacetimidoyl chlorides and their 1,3-dipolar cycloadditions with electron-deficient olefins" CHEM. LETT. (CMLTAG,03667022);1983; (9); PP.1463-4, XP002102463 Seikei Univ.;Dep. Ind. Chem.; Musashino; 180; Japan (JP)

## Description

### BACKGROUND OF THE INVENTION

2-Aryl-5-(perfluoroalkyl)pyrrole compounds are useful as insecticidal and acaricidal agents. In addition, those compounds are also useful for the preparation of other insecticidal and acaricidal agents. In particular, 2-aryl-5-(perfluoroalkyl)pyrrole compounds are key intermediates in the preparation of arylpyrrole compounds such as chlorfenapyr. Accordingly, there is an ongoing search to discover new methods for the preparation of 2-aryl-5-(perfluoralkyl)pyrrole compounds.

U.S. Pat. No. 5,145,986 discloses that 2-aryl-5-(trifluoromethyl)pyrrole compounds may be prepared from N-(substituted benzyl)-2,2,2-trifluoroacetimidoyl chloride compounds. U.S. Pat. Nos. 5,446,170 and 5,426,225 disclose that 2-aryl-5-(trifluoromethyl)pyrrole compounds may be obtained in several steps from the appropriate aldehyde. The processes described in U.S. Pat. Nos. 5,446,170 and 5,426,225 require the use of an aminonitrile intermediate which is obtained via the Strecker synthesis from the appropriate aldehyde. However, the use of the Strecker synthesis is not entirely satisfactory because of cyanide containing waste streams.

K. Tanaka et al., Chem. Lett., 1983, pp. 1463 f, disclose the isomerization of N-arylmethyltrifluoroacetimidoyl Chloride to N-(arylmethylene)-1-chloro-2,2,2-trifluoroethylamines in the presence of triethylamine. The reaction of trifluoroacetimidoyl chlorides with methyl acrylate or acrylonitril is also disclosed.

It is, therefore, an object of the present invention to provide a new process for the preparation of 2-aryl-5-(perfluoroalkyl)pyrrole compounds which avoids the use of the Strecker synthesis.

It is also an object of this invention to provide a new process for the preparation of arylpyrrole compounds such as chlorfenapyr.

A further object of the present invention is to provide new intermediate compounds which are useful in the processes described hereinbelow.

Those and other objects of the present invention will become more apparent from the detailed description thereof set forth below.

### SUMMARY OF THE INVENTION

The present invention provides a new process for the preparation of 2-aryl-5-(perfluoroalkyl)pyrrole compounds having the structural formula I 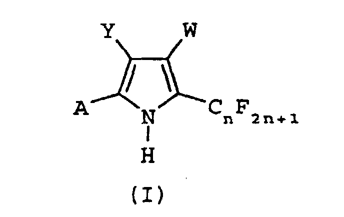 wherein
W is hydrogen;
Y is CN, NO₂ or CO₂R;
R is C₁-C₄alkyl;
m and n are each independently an integer of 1, 2, 3, 4, 5, 6, 7 or 8;
A is 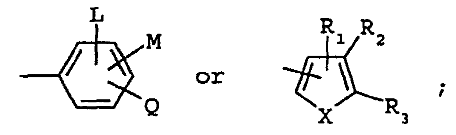 L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl or when M and Q are on adjacent positions they may be taken together with the carbon atoms to which they are attached to form a ring in which MQ represents the structure -OCH₂O-, -OCF₂O- or -CH=CH-CH=CH-;
R₁, R₂ and R₃ are each independently hydrogen, halogen, NO₂, CHO or R₂ and R₃ may be taken together with the atoms to which they are attached to form a ring in which R₂R₃ is represented by the structure 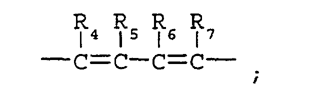 R₄, R₅, R₆ and R₇ are each independently hydrogen, halogen, CN or NO₂; and
X is O or S
which process comprises reacting an N-(arylmethylene)-1-chloro-1-(perfluoroalkyl)methylamine compound having the structural formula II 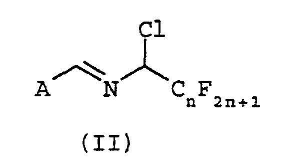 wherein A and n are as described above with a dieneophile compound having the structural formula III 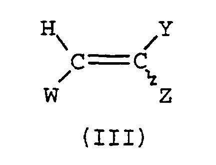 wherein W and Y are as described above and Z is Cl, Br or I, and a base in the presence of a solvent.

The present invention further provides novel N-(arylmethylene)-1-chloro-1-(perfluoroalkyl)methylamine compounds having the structural formula II 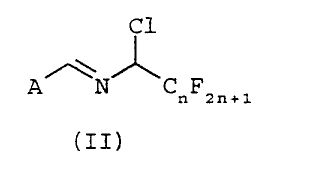 wherein n and A are as described hereinabove, provided that when A is unsubstituted phenyl, *p*-chlorophenyl or p-methylphenyl, n is an integer other than 1.

### DETAILED DESCRIPTION OF THE INVENTION

The process of the present invention preferably comprises reacting an N-(arylmethylene)-l-chloro-1-(perfluoroalkyl)methylamine compound of formula II with at least about one molar equivalent, preferably about one to four molar equivalents, of a dienophile compound of formula III and at least about one molar equivalent, preferably about one to four molar equivalents, of a base in the presence of a solvent preferably at a temperature range of about 5°C to 100°C to form 2-aryl-5-(perfluoroalkyl)pyrrole compounds of formula I.

Alternatively, the formula I compounds may be prepared by forming the formula III dienophile compounds *in situ.* This process comprises reacting an N-(arylmethylene ) -1-chloro-1- (perfluoroalkyl)methylamine compound of formula II with preferably about one to four molar equivalents of an α,β-dihalo compound having the structural formula IV 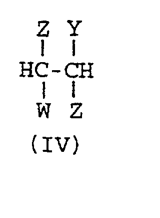 wherein W and Y are as described hereinabove and Z is Cl, Br or I, and at least about two molar equivalents, preferably about two to five molar equivalents, of a base in the presence of a solvent preferably at a temperature range of about 5°C to 100°C to form 2-aryl-5-(perfluoroalkyl)pyrrole compounds of formula I.

Advantageously, the present invention provides new processes for the preparation of 2-aryl-5-(perfluoroalkyl)pyrrole compounds which avoid the use of the Strecker synthesis.

The formula I compounds of this invention may be isolated by conventional procedures such as dilution of the reaction mixture with water and filtration or, alternatively, extraction with a suitable solvent. Suitable extraction solvents include water-immiscible solvents such as ether, ethyl acetate, toluene, methylene chloride and the like.

Bases suitable for use in this invention include tri-(C₁-C₆alkyl)amines such as trimethylamine, triethylamine, tripropylamine, tributylamine, diisopropylethylamine and the like; alkali metal carbonates such as potassium carbonate and sodium carbonate; alkali metal hydroxides such as potassium hydroxide and sodium hydroxide; alkali metal acetates such as potassium acetate and sodium acetate; and heterocyclic tertiary amines including, but not limited to, 1,8-diazabicyclo[5.4.0]undec-7-ene (DBU); 1,5-diazabicyclo[4.3.0]non-5-ene (DBN); 1,4-diazabicyclo[2.2.2]-octane; pyridine; substituted pyridines such as 2,6-dimethylpyridine, 2-methylpyridine, 3-methylpyridine, 4-methylpyridine and the like; quinoline; and substituted quinolines. Preferred bases include tri-(C₁-C₆alkyl)-amines, 1,8-diazabicyclo[5.4.0]undec-7-ene, 1,5-diazabicyclo[4.3.0]non-5-ene, 1,4-diazabicyclo[2.2.2]-octane, potassium carbonate and sodium carbonate.

Solvents suitable for use in the present invention include, but are not limited to, carboxylic acid amides such as N,N-dimethylformamide, N,N-dimethylacetamide and the like; N-substituted pyrrolidinones such as N-methylpyrrolidinone and the like; nitriles such as acetonitrile, propionitrile and the like; halogenated hydrocarbons such as methylene chloride, chloroform, carbon tetrachloride and the like; ethers such as tetrahydrofuran, dioxane and the like; sulfoxides such as dimethyl sulfoxide and the like; and mixtures thereof. Preferred solvents include carboxylic acid amides and nitriles and mixtures thereof. N,N-dimethylformamide and acetonitrile and mixtures thereof are especially preferred for use in the present invention.

Exemplary of halogen hereinabove are fluorine, chlorine, bromine and iodine. The terms "C₁-C₄haloalkyl", "C₁-C₄haloalkoxy", "C₁-C₄haloalkylthio", "C₁-C₄haloalkylsulfinyl" sulfinyl" and "C₁-C₄haloalkylsulfonyl" are defined as a C₁-C₄alkyl, C₁-C₄alkoxy, C₁-C₄alkylthio, C₁-C₄alkylsulfinyl or C₁-C₄alkylsulfonyl group substituted with one or more halogen atoms, respectively.

The present invention is especially useful for the preparation of formula I compounds wherein
W is hydrogen;
Y is CN;
n is 1 or 2;
A is 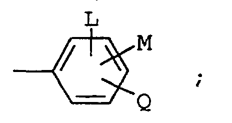 L is hydrogen or halogen; and
M and Q are each independently hydrogen, halogen, C₁-C₄haloalkyl or C₁-C₄haloalkoxy.

In particular, the present invention is useful for the preparation of
2-(*p*-chlorophenyl)-5-(trifluoromethyl)pyrrole-3-carbonitrile;
2-(3,5-dichlorophenyl)-5-(trifluoromethyl)pyrrole-3-carbonitrile;
2-(3,4-dichlorophenyl)-5-(trifluoromethyl)pyrrole-3-carbonitrile; and
2-[4-(trifluoromethyl) phenyl]-5-(trifluoromethyl)pyrrole-3-carbonitrile, among others.

The present invention also relates to novel N-(arylmethylene)-1-chloro-1-(perfluoroalkyl)methylamine compounds having the structural formula II 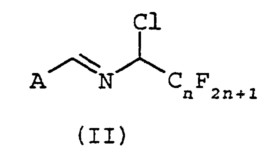 wherein
n is an integer of 1, 2, 3, 4, 5, 6, 7 or 8;
A is 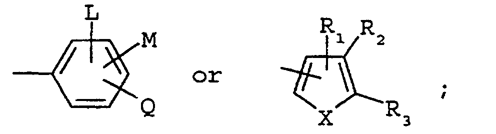 L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl or when M and Q are on adjacent positions they may be taken together with the carbon atoms to which they are attached to form a ring in which MQ represents the structure -OCH₂O-, -OCF₂O- or -CH=CH-CH=CH-;
R₁, R₂ and R₃ are each independently hydrogen, halogen, NO₂, CHO or R₂ and R₃ may be taken together with the atoms to which they are attached to form a ring in which R₂R₃ is represented by the structure 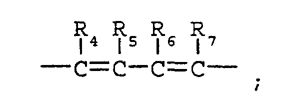 R₄, R₅, R₆ and R₇ are each independently hydrogen, halogen, CN or NO₂; and
X is O or S,
provided that when A is unsubstituted phenyl, p-chlorophenyl or p-methylphenyl, n is an integer other than 1.

Preferred novel formula II compounds of this invention are those wherein
n is 1 or 2;
A is 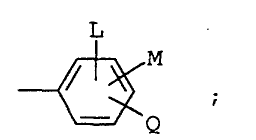 L is hydrogen or halogen; and
M and Q are each independently halogen, C₁-C₄haloalkyl or C₁-C₄haloalkoxy.

Novel formula II compounds which are particularly useful in the processes of this invention include
1-chloro-N-(3,5-dichlorobenzylidine)-2,2,2-trifluoroethylamine;
1-chloro-N-(3,4-dichlorobenzylidine)-2,2,2-trifluoroethylamine; and
1-chloro-2,2,2-trifluoro-N-[4-(trifluoromethyl)-benzylidine]ethylamine, among others.

Starting N-(arylmethylene)-1-chloro-1-(perfluoroalkyl)methylamine compounds of formula II may be prepared, as shown in Flow Diagram I, by isomerizing an N-(arylmethyl)perfluoroalkylformimidoyl chloride compound having the structural formula V with a tertiary amine optionally at an elevated temperature according to the procedures described in Chemistry Letters, pp. 1463-1464 (1983).

### FLOW DIAGRAM I

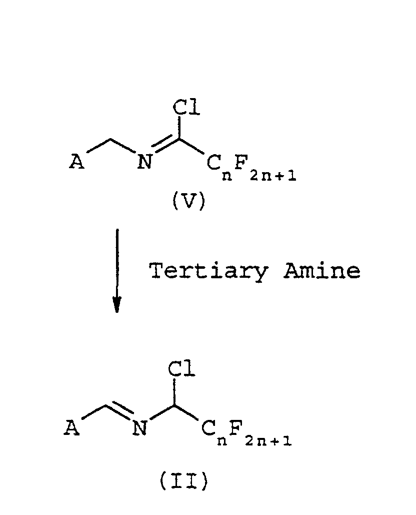

Tertiary amines suitable for use to isomerize the formula V compounds include tri-(C₁-C₆alkyl)amines such as triethylamine, tripropylamine, diisopropylethylamine and the like; and heterocyclic tertiary amines including, but not limited to, 1,8-diazabicyclo[5.4.0]undec-7-ene; 1,5-diazabicyclo[4.3.0]non-5-ene; pyridine; substituted pyridines such as 2,6-dimethylpyridine, 2-methylpyridine, 3-methylpyridine, 4-methylpyridine and the like; quinoline; and substituted quinolines. Preferred tertiary amines include tri-(C₁-C₆alkyl) amines, 1,8-diazabicyclo[5.4.0]undec-7-ene and 1,5-diazabicyclo-[4.3. O]non-5-ene.

In a preferred embodiment of the present invention, the formula V compound is isomerized with a tertiary amine in the presence of a solvent at a temperature range of about 15°C to 60°C. Solvents suitable for use in the preparation of the formula II compounds include aromatic hydrocarbons such as toluene, benzene, xylenes and the like and mixtures thereof.

The N-(arylmethyl)perfluroalkylformimidoyl chloride compounds of formula V may be prepared according to the procedures described in U.S. Pat. No. 5,145,986 and Chemistry Letters, pp. 1463-1464 (1983).

Starting formula III dienophile compounds are known in the art and may be prepared using conventional procedures. Compounds of formulas III and IV wherein W is CₘF₂ₘ₊₁ may be prepared according to the procedures described in U.S. Pat. No. 5,068,390.

The formula I compounds are useful for the control of insect and acarid pests. In addition, the formula I compounds may be used to prepare other arylpyrrole insecticidal and acaricidal agents having the structural formula VI 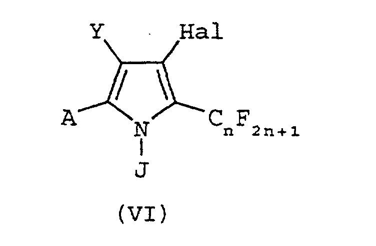 wherein
Y is CN, NO₂ or CO₂R;
R is C₁-C₄alkyl;
n is an integer of 1, 2, 3, 4, 5, 6, 7 or 8;
A is 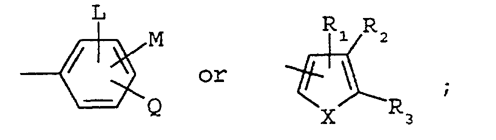 L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl or when M and Q are on adjacent positions they may be taken together with the carbon atoms to which they are attached to form a ring in which MQ represents the structure -OCH₂O-, -OCF₂O- or -CH=CH-CH=CH-;
R₁, R₂ and R₃ are each independently hydrogen, halogen, NO₂, CHO or R₂ and R₃ may be taken together with the atoms to which they are attached to form a ring in which R₂R₃ is represented by the structure 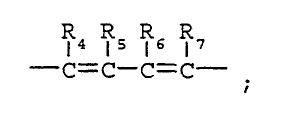 R₄, R₅, R₆ and R₇ are each independently hydrogen, halogen, CN or NO₂;
X is O or S;
Hal is a halogen atom; and
J is hydrogen or C₁-C₆alkoxymethyl.

The present invention is especially useful for the preparation of arylpyrrole compounds of formula VI wherein
Y is CN;
n is 1 or 2;
A is 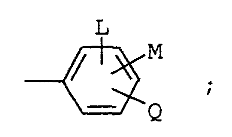 L is hydrogen or halogen;
M and Q are each independently hydrogen, halogen, C₁-C₄haloalkyl or C₁-C₄haloalkoxy;
Hal is Br or Cl; and
J is hydrogen or ethoxymethyl.

In particular, the present invention is useful for the preparation of formula VI arylpyrrole compounds such as
4-bromo-2-(*p*-chlorophenyl)-1-(ethoxymethyl)-5-(trifluoromethyl)pyrrole-3-carbonitrile (chlorfenapyr) ;
4-bromo-2-(3,4-dichlorophenyl)-1-(ethoxymethyl)-5-(trifluoromethyl)pyrrole-3-carbonitrile; and
4-bromo-2-(*p*-chlorophenyl)-5-(trifluoromethyl)pyrrole-3-carbonitrile, among others.

Advantageously, formula VI arylpyrrole compounds may be prepared by a process which comprises:
(a) reacting an N-(arylmethylene )-1-chloro-1-(perfluoroalkyl)methylamine compound of formula II with a dienophile compound having the structural formula VII 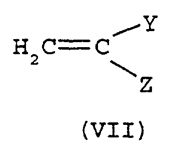 wherein Y is as described above and Z is Cl, Br or I, and a base in the presence of a solvent to form a 2-aryl-5-(perfluoroalkyl)pyrrole compound having the structural formula VIII 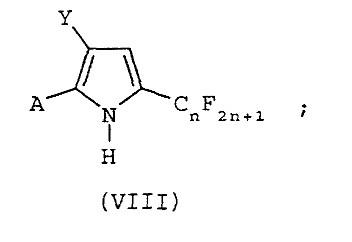
(b) halogenating the formula VIII compound to form the arylpyrrole compound of formula VI wherein J is hydrogen; and
(c) optionally alkoxymethylating the formula VI compound wherein J is hydrogen to form the formula VI arylpyrrole compound wherein J is C₁-C₆alkoxymethyl.

Alternatively, arylpyrrole compounds of formula VI may be prepared by a process which comprises:
(a) reacting an N-(arylmethylene)-1-chloro-1-(perfluoroalkyl)methylamine compound of formula II with an α,β-dihalo compound having the structural formula IX 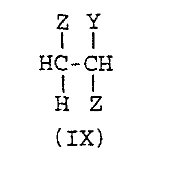 wherein Y is as described above and Z is Cl, Br or I, and at least about two molar equivalents of a base in the presence of a solvent to form a 2-aryl-5-(perfluoroalkyl)pyrrole compound having the structural formula VIII 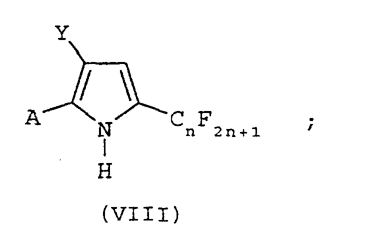
(b) halogenating the formula VIII compound to form the arylpyrrole compound of formula VI wherein J is hydrogen; and
(c) optionally alkoxymethylating the formula VI compound wherein J is hydrogen to form the formula VI arylpyrrole compound wherein J is C₁-C₆alkoxymethyl.

Halogenation methods may be any known methods such as those described in U.S. Pat. Nos. 5,010,098 and 5,449,789.

Alkoxymethylation procedures suitable for use in this invention include conventional procedures known in the art (see, e.g., U.S. Pat. Nos. 5,010,098 and 5,359,090). In a preferred embodiment of this invention, the alkoxymethylation procedure comprises reacting a formula VI compound wherein J is hydrogen with a di-(C₁-C₆alkoxy)methane compound, N,N-dimethylformamide and phosphorous oxychloride in the presence of an aprotic solvent to form a reaction mixture and treating the reaction mixture with a tertiary amine.

In order to facilitate a further understanding of this invention, the following examples are presented primarily for the purpose of illustrating more specific details thereof. The scope of the invention should not be deemed limited by the examples, but encompasses the entire subject matter defined in the claims.

### EXAMPLE 1

### Preparation of 1-Chloro-N-(p-chlorobenzylidine) 2,2,2-trifluoroethylamine

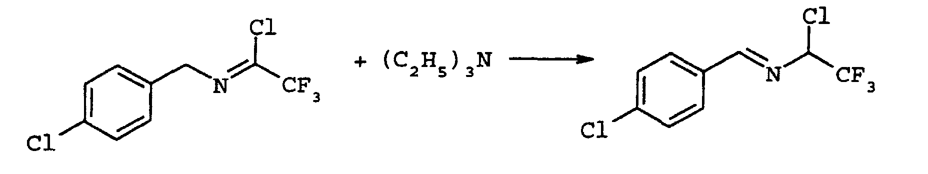

A solution of N-(p-chlorobenzyl)-2,2,2-trifluoroacetimidoyl chloride (102.4 g, 0.4 mol) in toluene is treated with triethylamine (40.5 g, 0.4 mol) over 45 minutes as the temperature rises to 40°C, stirred for 18 hours, and filtered to remove solids. The filtrate is concentrated *in vacuo* to remove toluene and triethylamine, and the resultant residue is vacuum distilled to give the title product as a colorless liquid (87.0 g, 87% yield, bp 95-98°C/2.6 mmHg =̂ 347 Pa).

Using essentially the same procedure, but using the appropriately substituted acetimidoyl chloride, the following compounds are obtained:

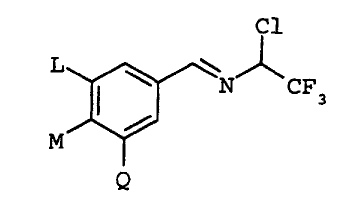

| **L** | **M** | **Q** | **bp°C/mmHg(=̂ 133.3 Pa)** | **%Yield** |
|---|---|---|---|---|
| H | Cl | Cl | 101-104/0.5(67) | 79 |
| H | CF₃ | H | | |
| Cl | H | Cl | 110-112/0.9(120) | 84 |

### EXAMPLE 2

### Preparation of 2-(p-Chlorophenyl)-5-(trifluoro-methyl)pyrrole-3-carbonitrile

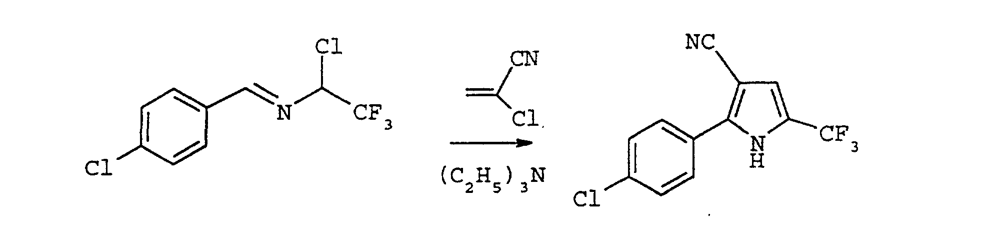

A solution of 1-chloro-N-(p-chloro-benzylidine)-2,2,2-trifluoroethylamine (9.0 g, 0.035 mol) and 2-chloroacrylonitrile (3.7 g, 0.042 mol) in N,N-dimethylformamide is treated dropwise with triethylamine (7.8 g, 0.077 mol) over 45 minutes as the temperature rises to 45°C, stirred at 55-60°C for 4 hours, stirred overnight at room temperature, and diluted with water and ethyl acetate. The organic layer is separated, washed with water, and concentrated *in vacuo* to obtain a residue. Flash column chromatography of the residue using silica gel and a 20% ethyl acetate in heptane solution gives the title product as a pale yellow solid (3.9 g, 41.2% yield) which is identified by ¹H and ¹⁹F NMR.

Following essentially the same procedure, but using the appropriately substituted 1-chloro-2,2,2-trifluoro-N-(substituted benzylidine)ethylamine, the following compounds are obtained:

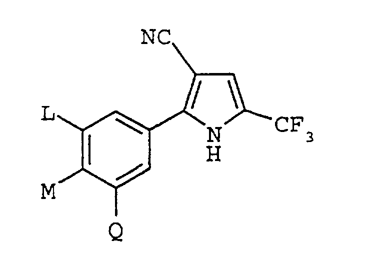

| **L** | **M** | **Q** | **mp°C** |
|---|---|---|---|
| H | CF₃ | H | 216-217.5 |
| Cl | H | Cl | |

### EXAMPLE 3

### Preparation of 2- (3,4-Dichlorophenyl) -5- (trifluoromethyl)pyrrole-3-carbonitrile using 2-chloroacrylonitrile

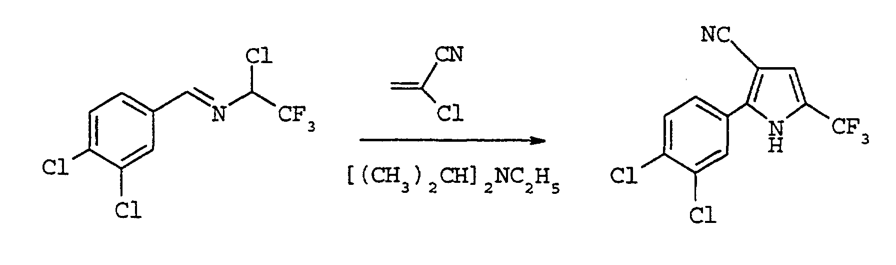

A solution of 1-chloro-N-(3,4-dichlorobenzylidine)-2,2,2-trifluoroethylamine (11.6 g, 0.04 mol) and 2-chloroacrylonitrile (4.2 g, 0.048 mol) in N,N-dimethylformamide is treated dropwise with diisopropylethylamine (12.9 g, 0.1 mol) over 45 minutes as the temperature rises to 40°C, stirred at 55°C for 4 hours, stirred overnight at room temperature, and diluted with water and ethyl acetate. The organic layer is separated, washed with water, and concentrated *in vacuo* to obtain a residue. Flash column chromatography of the residue using silica gel and a 20% ethyl acetate in heptane solution gives the title product as a white solid (5.8 g, 47.5% yield) which is identified by ¹H and ¹⁹F NMR.

### EXAMPLE 4

### Preparation of 2 -(3,4-Dichlorophenyl)-5 -(trifluoromethyl)pyrrole-3-carbonitrile using 2,3-dichloropropionitrile

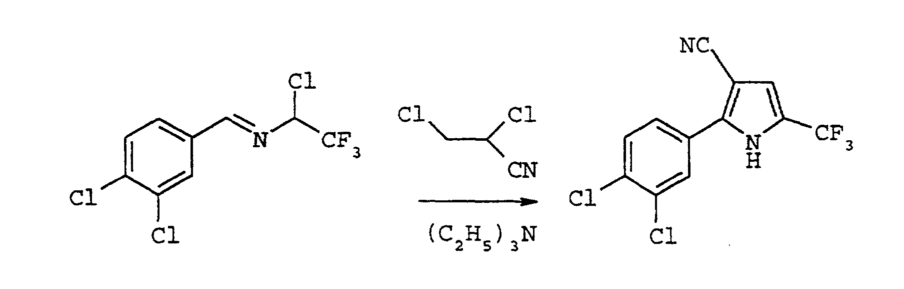

A solution of 1-chloro-N-(3,4-dichlorobenzylidine)-2,2,2-trifluoroethylamine (8.72 g, 0.03 mol) and 2,3-dichloropropionitrile (4.3 g, 0.0345 mol) in N,N-dimethylformamide is treated dropwise with triethylamine (11.1 g, 0.11 mol) over 30 minutes as the temperature rises to 55°C, stirred at 55°C for 5 hours, cooled to room temperature, and diluted with water and ethyl acetate. The organic layer is separated, washed with water, and concentrated in *vacuo* to obtain a residue. The residue is dissolved in ethyl acetate and mixed with silica gel. The silica gel mixture is dried *in* vacuo and washed with a 20% ethyl acetate in heptane solution. The resultant filtrate is concentrated in *vacuo* and the residue is triturated with heptane and a small amount of ethyl acetate to give the title product as a pale yellow solid (3.2 g, 35% yield).

## Claims

1. A process for the preparation of a 2-aryl-5-(perfluoroalkyl)pyrrole compound having the structural formula I 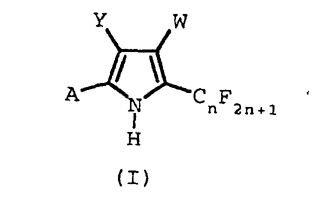 wherein
W is hydrogen;
Y is CN, NO₂ or CO₂R;
R is C₁-C₄alkyl;
m and n are each independently an integer of 1, 2, 3, 4, 5, 6, 7 or 8;
A is 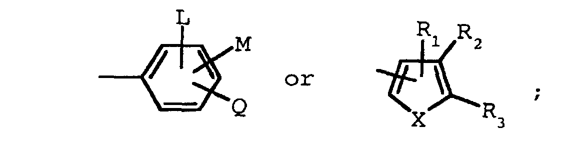 L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₁-C₄haloalkylthio, C₁-C₄alkylsulfinyl, C₁-C₄haloalkylsulfinyl,C₁-C₄alkylsulfonyl, C₁-C₄haloalkylsulfonyl or when M and Q are on adjacent positions they may be taken together with the carbon atoms to which they are attached to form a ring in which MQ represents the structure -OCH₂O-, -OCF₂O- or -CH=CH-CH=CH-;
R₁, R₂ and R₃ are each independently hydrogen, halogen, NO₂, CHO or R₂ and R₃ may be taken together with the atoms to which they are attached to form a ring in which R₂R₃ is represented by the structure 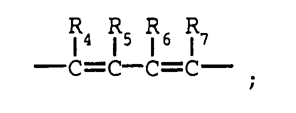 R₄, R₅, R₆ and R₇ are each independently hydrogen, halogen, CN or NO₂;
and
X is 0 or S
which process comprises reacting an N-(arylmethylene)-1-chloro-l-(perfluoroalkyl)methylamine compound having the structural formula II 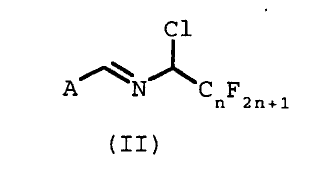 wherein A and n are as described above with a dieneophile compound having the structural formula III or an α,β-dihalo compound having the structural formula IV 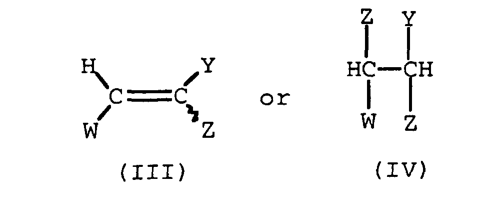 wherein W and Y are as described above and Z is Cₗ, Br or I, and a base in the presence of a solvent.

2. The process according to claim 1 wherein the base is selected from the group consisting of a tri(C₁-C₆alkyl)amine, an alkali metal carbonate and a heterocyclic tertiary amine.

3. The process according to claim 1 wherein the solvent is selected from the group consisting of a carboxylic acid amide and a nitrile and mixtures thereof.

4. The process according to claim 1 wherein the dieneophile is present in the amount of about one to four molar equivalents and the base is present in the amount of about one to four molar equivalents.

5. The process according to claim 1 wherein the α,β-dihalo compound is present in the amount of about one to four molar equivalents and the base is present in the amount of about two to five molar equivalents.

6. The process according to claim 1 wherein
W is hydrogen;
Y is CN;
n is 1 or 2;
A is 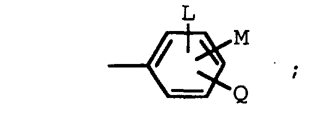 L is hydrogen or halogen; and
M and Q are each independently hydrogen, halogen, C₁-C₄haloalkyl or C₁-C₄haloalkoxy.

7. A process for the preparation of an arylpyrrole compound having the structural formula VI 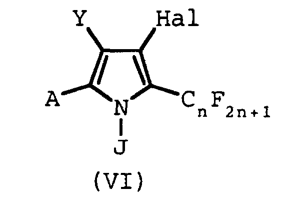 wherein Y, n and A are as defined in claim 1,
Hal is a halogen atom; and
J is hydrogen or C₁-C₆alkoxymethyl
which process comprises the steps of:
(a) preparing a compound of the formula I, wherein W is hydrogen, by a process according to claim 1, which comprises reacting a compound of the formula II as defined in claim 1 with a compound of the formulae III or IV as defined in claim 1, wherein W is hydrogen;
(b) halogenating the formula I compound, wherein W is hydrogen, to form the arylpyrrole compound of formula VI wherein J is hydrogen; and
(c) optionally alkoxymethylating the formula VI compound wherein J is hydrogen to obtain a compound of the formula VI, wherein J is C₁-C₆alkoxymethyl.

8. The process according to claim 7 wherein step (c) comprises reacting the formula VI compound wherein J is hydrogen with a di-(C₁-C₆alkoxy)methane compound, N,N-dimethylformamide and phosphorous oxychloride in the presence of an aprotic solvent to form a reaction mixture and treating the reaction mixture with a tertiary amine.

9. A compound having the structural formula II 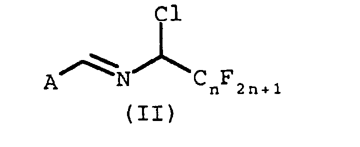 wherein
n is an integer of 1, 2, 3, 4, 5, 6, 7 or 8;
A is 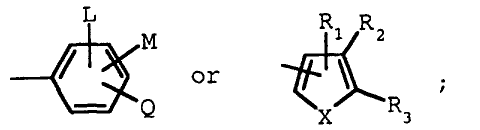 L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl or when M and Q are on adjacent positions they may be taken together with the carbon atoms to which they are attached to form a ring in which MQ represents the structure -OCH₂O-, -OCF₂O- or -CH=CH-CH=CH-
R₁, R₂ and R₃ are each independently hydrogen, halogen, NO₂, CHO or R₂ and R₃ may be taken together with the atoms to which they are attached to form a ring in which R₂R₃ is represented by the structure 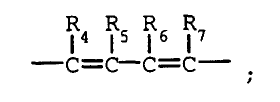 R₄, R₅, R₆ and R₇ are each independently hydrogen, halogen, CN or NO₂;
and
X is O or S,
provided that when A is unsubstituted phenyl, p-chlorophenyl or p-methylphenyl, n is an integer other than 1.

10. The compound according to claim 9 wherein
n is 1 or 2;
A is 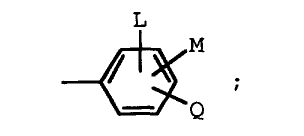 L is hydrogen or halogen; and
M and Q are each independently hydrogen, halogen, C₁-C₄haloalkyl or C₁-C₄haloalkoxy.

## Patentansprüche

1. Verfahren zur Herstellung einer 2-Aryl-5-(perfluoralkyl)pyrrol-Verbindung mit der Strukturformel I 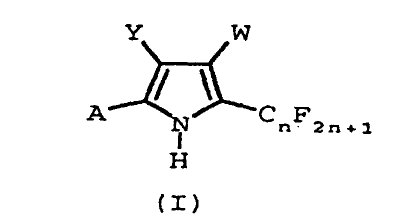 worin
W für Wasserstoff steht;
Y für CN, NO₂ oder CO₂R steht;
R für (C₁-C₄)-Alkyl steht;
m und n jeweils unabhängig voneinander für die ganze Zahl 1, 2, 3, 4, 5, 6, 7 oder 8 stehen;
A für 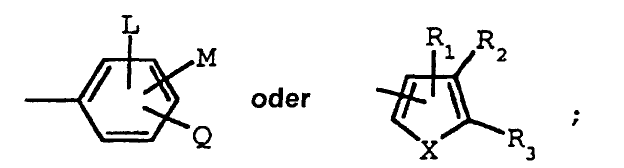 steht;
L für Wasserstoff oder Halogen steht;
M und Q jeweils unabhängig voneinander für Wasserstoff, Halogen, CN, NO₂, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfinyl, (C₁-C₄)-Halogenalkylsulfinyl, (C₁-C₄)-Alkylsulfonyl, (C₁-C₄)-Halogenalkylsulfonyl stehen oder M und Q, wenn sie einander benachbart sind, zusammen mit den Kohlenstoffatomen, mit denen sie verknüpft sind, einen Ring bilden können, bei dem MQ die Struktur -OCH₂O-, -OCF₂O- oder -CH=CH-CH=CH- darstellt;
R₁, R₂ und R₃ jeweils unabhängig voneinander für Wasserstoff, Halogen, NO₂, CHO stehen oder R₂ und R₃ zusammen mit den Atomen, mit denen sie verknüpft sind, einen Ring bilden können, bei dem R₂R₃ durch die Struktur 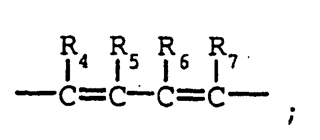 gegeben ist;
R₄, R₅, R₆ und R₇ jeweils unabhängig voneinander für Wasserstoff, Halogen, CN oder NO₂ stehen;
und
X für O oder S steht,
wobei man eine N-(Arylmethylen)-1-chlor-1-(perfluoralkyl)methylamin-Verbindung mit der Strukturformel II 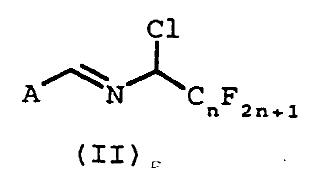 worin A und n wie oben beschrieben definiert sind, mit einer dienophilen Verbindung mit der Strukturformel III oder einer α,β-Dihalogen-Verbindung mit der Strukturformel IV 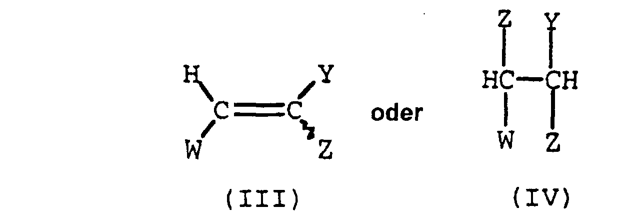 worin W und Y wie oben beschrieben definiert sind und Z für C1, Br oder I steht, und einer Base in Gegenwart eines Lösungsmittels umsetzt.

2. Verfahren nach Anspruch 1, wobei die Base ausgewählt ist unter einem Tri((C₁-C₆)-alkyl)amin, einem Alkalimetallcarbonat und einem heterozyklischen tertiären Amin.

3. Verfahren nach Anspruch 1, wobei das Lösungsmittel ausgewählt ist unter einem Carbonsäureamid und einem Nitril und Mischungen davon.

4. Verfahren nach Anspruch 1, wobei das Dienophil in einer Menge von etwa ein bis vier molaren Äquivalenten vorhanden ist und die Base in einer Menge von etwa ein bis vier molaren Äquivalenten vorhanden ist.

5. Verfahren nach Anspruch 1, wobei die α,β-Dihalogen-Verbindung in einer Menge von etwa ein bis vier molaren Äquivalenten vorhanden ist und die Base in einer Menge von etwa zwei bis fünf molaren Äquivalenten vorhanden ist.

6. Verfahren nach Anspruch 1, worin
W für Wasserstoff steht;
Y für CN steht;
n für 1 oder 3 steht;
A für 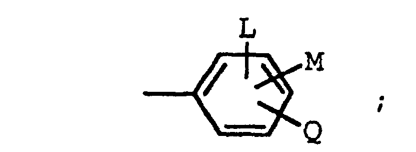 steht;
L für Wasserstoff oder Halogen steht; und
M und Q jeweils unabhängig voneinander für Wasserstoff, Halogen, (C₁-C₄)-Halogenalkyl oder (C₁-C₄)-Halogenalkoxy stehen.

7. Verfahren zur Herstellung einer Arylpyrrol-Verbindung mit der Strukturformel VI 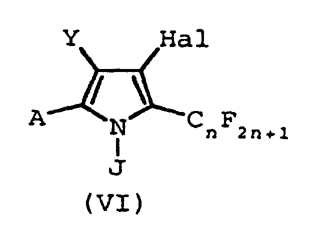 worin Y, n und A wie in Anspruch 1 definiert sind,
Hal für ein Halogenatom steht; und
J für Wasserstoff oder (C₁-C₆)-Alkoxymethyl steht,
umfassend die Schritte:
a) Herstellen einer Verbindung der Formel I, worin W für Wasserstoff steht, durch ein Verfahren nach Anspruch 1, wobei man eine Verbindung der Formel II, wie in Anspruch 1 definiert, mit einer Verbindung der Formeln III oder IV, wie in Anspruch 1 definiert, worin W für Wasserstoff steht, umsetzt;
b) Halogenieren der Verbindung der Formel I, worin W für Wasserstoff steht, so dass die Arylpyrrol-Verbindung der Formel VI gebildet wird, worin J für Wasserstoff steht; und
c) gegebenenfalls Alkoxymethylieren der Verbindung der Formel VI, worin J für Wasserstoff steht, so dass eine Verbindung der Formel VI erhalten wird, worin J für (C₁-C₆)-Alkoxymethyl steht.

8. Verfahren nach Anspruch 7, wobei Schritt c) das Umsetzen der Verbindung der Formel VI, worin J für Wasserstoff steht, mit einer Di-((C₁-C₆)-alkoxy)methan-Verbindung, N.N-Dimethylformamid und Phosphortrichloridoxid in Gegenwart eines aprotischen Lösungsmittels unter Bildung einer Reaktionsmischung und Behandlung der Reaktionsmischung mit einem tertiären Amin umfasst.

9. Verbindung mit der Strukturformel II 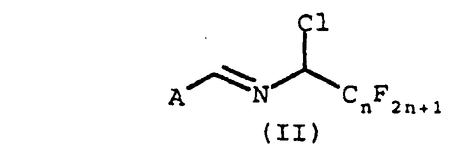 worin
n für die ganze Zahl 1, 2, 3, 4, 5, 6, 7 oder 8 steht;
A für 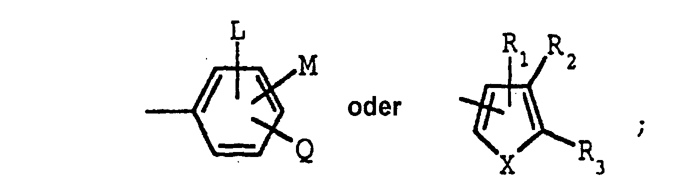 steht;
L für Wasserstoff oder Halogen steht;
M und Q jeweils unabhängig voneinander für Wasserstoff, Halogen, CN, NO₂, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, (C₁-C₄)-Halogenalkylthio, (C₁-C₄)-Alkylsulfinyl, (C₁-C₄)-Halogenalkylsulfinyl, (C₁-C₄)-Alkylsulfonyl, (C₁-C₄)-Halogenalkylsulfonyl stehen oder M und Q, wenn sie einander benachbart sind, zusammen mit den Kohlenstoffatomen, mit denen sie verknüpft sind, einen Ring bilden können, bei dem MQ die Struktur - OCH₂O-, -OCF₂O- oder -CH=CH-CH=CH- darstellt;
R₁, R₂ und R₃ jeweils unabhängig voneinander für Wasserstoff, Halogen, NO₂, CHO stehen oder R₂ und R₃ zusammen mit den Atomen, mit denen sie verknüpft sind, einen Ring bilden können, bei dem R₂R₃ durch die Struktur 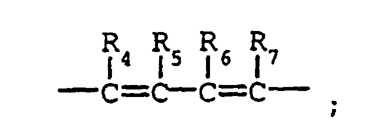 gegeben ist;
R₄, R₅, R₆ und R₇ jeweils unabhängig voneinander für Wasserstoff, Halogen, CN oder NO₂ stehen;
und
X für 0 oder S steht,
mit der Maßgabe, dass n für eine von eins verschiedene ganze Zahl steht, wenn A für unsubstituiertes Phenyl, p-Chlorphenyl oder p-Methylphenyl steht.

10. Verbindung nach Anspruch 9, worin
n für 1 oder 2 steht;
A für 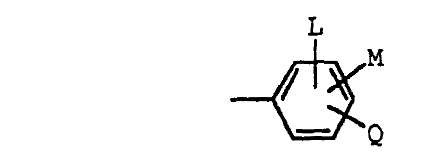 steht;
L für Wasserstoff oder Halogen steht; und
M und Q jeweils unabhängig voneinander für Wasserstoff, Halogen, (C₁-C₄)-Halogenalkyl oder (C₁-C₄)-Halogenalkoxy stehen.

## Revendications

1. Procédé pour la préparation d'un dérivé de 2-aryl-5-(perfluoroalkyl)pyrrole répondant à la formule développée l 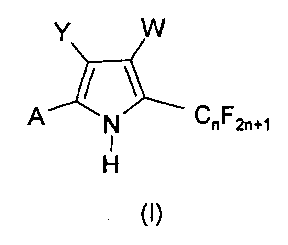 dans laquelle
W représente un atome d'hydrogène ;
Y représente CN, NO₂ ou CO₂R;
R représente un groupe alkyle en C₁-C₄;
m et n représentent chacun indépendamment un nombre entier de 1, 2, 3, 4, 5, 6, 7 ou 8 ;
A représente 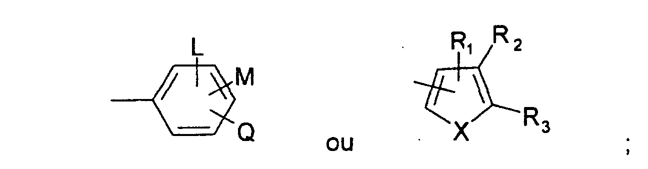 L représente un atome d'hydrogène ou un atome d'halogène ;
M et Q représentent chacun indépendamment un atome d'hydrogène, un atome d'halogène, CN, NO₂, un groupe alkyle en C₁-C₄, un groupe halogénoalkyle en C₁-C₄, un groupe alcoxy en C₁-C₄, un groupe halogénoalcoxy en C₁-C₄, un groupe alkylthio en C₁-C₄, un groupe halogénoalkylthio en C₁-C₄, un groupe alkylsulfinyle en C₁-C₄, un groupe halogénoalkylsulfinyle en C₁-C₄, un groupe alkylsuffonyle en C₁-C₄, un groupe halogénoalkylsulfonyle en C₁-C₄ ou quand M et Q sont en positions adjacentes, ils peuvent être pris ensemble avec les atomes de carbone auxquels ils sont attachés pour former un cycle dans lequel MQ représente la structure -OCH₂O-, -OCF₂O- ou -CH=CH-CH=CH-;
R₁, R₂ et R₃ représentent chacun indépendamment un atome d'hydrogène, un atome d'halogène, NO₂, CHO ou R₂ et R₃ peuvent être pris ensemble avec les atomes auxquels ils sont attachés pour former un cycle dans lequel R₂R₃ est représenté par la structure 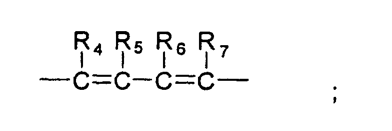 R₄, R₅, R₆ et R₇ représentent chacun indépendamment un atome d'hydrogène, un atome d'halogène, CN ou NO₂ ;
et
X représente O ou S,
lequel procédé comprend la réaction d'un dérivé de N-(arylméthylène)-1-chloro-1-(perfluoroalkyl)méthylamine répondant à la formule développée II 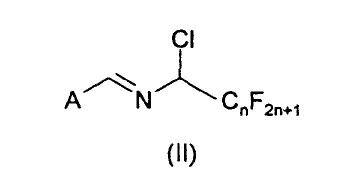 dans laquelle A et n sont tels que décrits ci-dessus avec un composé diéneophile répondant à la formule développée III ou un composé α,β-dihalogéné répondant à la formule développée IV 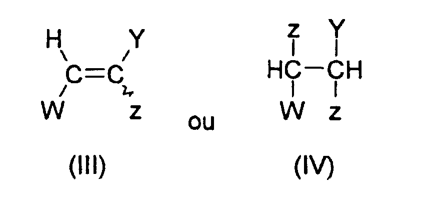 dans laquelle W et Y sontteisque décrits ci-dessus et Z représente CI, Br ou I, et d'une base en présence d'un solvant.

2. Procédé selon la revendication 1, dans lequel la base est choisie dans le groupe comprenant une tri(alkyle en C₁-C₆)amine, un carbonate de métal alcalin et une aminé tertiaire hétérocyclique.

3. Procédé selon la revendication 1, dans lequel le solvant est choisi parmi le groupe comprenant un amide d'un acide carboxylique et un nitrile et des mélanges de ceux-ci.

4. Procédé selon la revendication 1, dans lequel le diéneophile est présent en la quantité comprise dans la plage allant d'environ un à quatre équivalents molaires et la base est présente en la quantité comprise dans la plage allant d'environ un à quatre équivalents molaires.

5. Procédé selon la revendication 1, dans lequel le composé α,β-dihalogéné est présent en la quantité comprise dans la plage allant d'environ un à quatre équivalents molaires et la base est présente en la quantité comprise dans la plage allant d'environ deux à cinq équivalents molaires.

6. Procédé selon la revendication 1, dans lequel
W représente un atome d'hydrogène ;
Y représente CN ;
n vaut 1 ou 2 ;
A représente 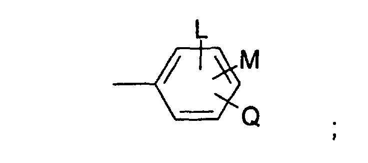 L est un atome d'hydrogène ou un atome d'halogène ; et
M et Q représentent chacun indépendamment un atome d'hydrogène, un atome d'halogène, un groupe halogénoalkyle en C₁-C₄ ou un groupe halogénoalcoxy en C₁-C₄.

7. Procédé pour la préparation d'un dérivé d'arylpyrrole répondant à la formule développée VI 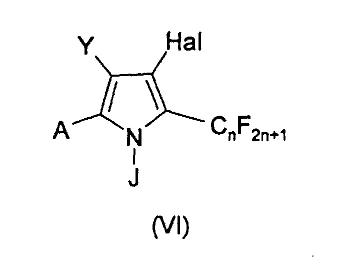 dans laquelle Y, n et A sont tels que définis en revendication 1,
Hal est un atome d'halogène ; et
J est un atome d'hydrogène ou un groupe alcoxyméthyle en C₁-C₆,
lequel procédé comprend les étapes consistant à :
(a) préparer un composé répondant à la formuJe l, dans laquelle W est un atome d'hydrogène, par un procédé selon la revendication 1, qui comprend la réaction d'un composé de formule II tel que défini en revendication 1, avec un composé de formule III ou IV tel que défini en revendication 1, dans laquelle W est un atome d'hydrogène ;
(b) halogéner le composé de formule 1, dans laquelle W est un atome d'hydrogène, pour former le dérivé d'arylpyrrole répondant à la formule VI dans laquelle J est un atome d'hydrogène ; et
(c) éventuellement alcoxyméthyler le composé de formule VI dans laquelle J est un atome d'hydrogène pour obtenir un composé répondant à la formule VI, dans laquelle J est un groupe alcoxyméthyle en C₁-C₆.

8. Procédé selon la revendication 7, dans lequel l'étape (c) comprend la réaction du composé de formule VI dans laquelle J est un atome d'hydrogène avec un dérivé de di-(alcoxy en C₁-C₆)méthane, du N,N-diméthylformamide et de l'oxychlorure de phosphore en présence d'un solvant aprotique pour former un mélange réactionnel et le traitement du mélange réactionnel avec une aminé tertiaire.

9. Composé répondant à la formule développée II 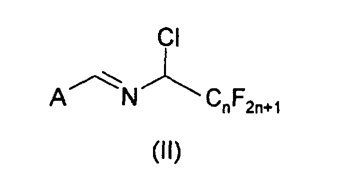 dans laquelle
n représente un nombre entier de 1, 2, 3, 4, 5, 6, 7 ou 8 ;
A représente 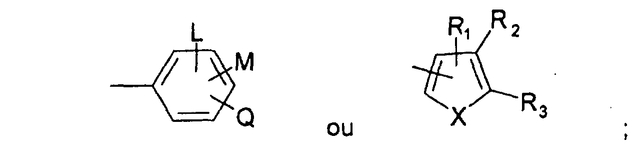 L représente un atome d'hydrogène ou un atome d'halogène ;
M et Q représentent chacun indépendamment un atome d'hydrogène, un atome d'halogène, CN, NO₂, un groupe alkyle en C₁-C₄, un groupe halogénoalkyle en C₁-C₄, un groupe alcoxy en C₁-C₄, un groupe halogénoalcoxy en C₁-C₄, un groupe alkylthio en C₁-C₄, un groupe halogénoalkylthio en C₁-C₄, un groupe alkylsulfinyle en C₁-C₄, un groupe halogénoalkylsulfinyle en C₁-C₄, un groupe alkylsulfonyle en C₁-C₄, un groupe halogénoalkylsulfonyle en C₁-C₄ ou quand M et Q sont en positions adjacentes, ils peuvent être pris ensemble avec les atomes de carbone auxquels ils sont attachés pour former un cycle dans lequel MQ représente la structure -OCH₂O-, -OCF₂O- ou -CH=CH-CH=CH-;
R₁, R₂ et R₃ représentent chacun indépendamment un atome d'hydrogène, un atome d'halogène, NO₂, CHO ou R₂ et R₃ peuvent être pris ensemble avec les atomes auxquels ils sont attachés pour former un cycle dans lequel R₂R₃ est représenté par la structure 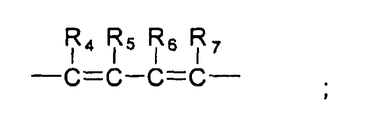 R₄, R₅, R₆ et R₇ représentent chacun indépendamment un atome d'hydrogène, un atome d'halogène, CN ou NO₂ ;
et
X représente O ou S,
à condition que quand A est un groupe phényle non substitué, un groupe p-chlorophényle ou un groupe p-méthylphényle, n est un nombre entier différent de 1.

10. Composé selon la revendication 9, dans lequel
n vaut 1 ou 2 ;
A représente 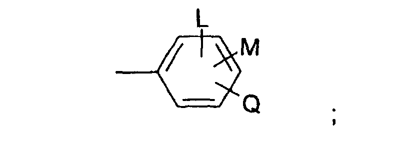 L est un atome d'hydrogène ou un atome d'halogène ; et
M et Q représentent chacun indépendamment un atome d'hydrogène, un atome d'halogène, un groupe halogénoalkyle en C₁-C₄ ou un groupe halogénoalcoxy en C₁-C₄.
